# EUROPEAN PATENT SPECIFICATION

(11) **EP 2 793 865 B1**
(45) Date of publication and mention of the grant of the patent: **31.01.2018**
(21) Application number: 12860701.7
(22) Date of filing: 18.12.2012
(51) Int. Cl.: A61K 9/50, A61K 38/09, A61P 5/04, A61P 15/08, A61P 35/00, A61K 9/16

(54) **PHARMACEUTICAL COMPOSITIONS OF TRIPTORELIN MICROSPHERES**
PHARMAZEUTISCHE ZUSAMMENSETZUNGEN AUS TRIPTORELIN-MIKROKUGELN
COMPOSITIONS PHARMACEUTIQUES DE MICROSPHÈRES DE TRIPTORÉLINE

(30) Priority: 22.12.2011 CN 201110435894
(43) Date of publication of application: 29.10.2014
(73) Proprietor: SHANDONG LUYE PHARMACEUTICAL CO., LTD., Yantai, Shandong 264003 (CN)
(72) Inventor: SUN, Kaoxiang, Yantai Shandong 264003 (CN); SONG, Tao, Yantai Shandong 264003 (CN); WANG,Qilin, Yantai Shandong 264003 (CN); HAN, Jie, Yantai Shandong 264003 (CN); WANG, Tao, Yantai Shandong 264000 (CN); HAN, Jiangbin, Yantai Shandong 264003 (CN); ZHANG, Jianzhao, Yantai Shandong 264003 (CN); WANG, Shujiang, Yantai Shandong 26119 (CN)
(74) Representative: KNH Patentanwälte
(86) International application number: PCT/CN2012/001712
(87) International publication number: WO 2013/091283

(56) References cited:
- WO-A1-2004/112752
- CN-A- 1 876 173
- CN-A- 101 627 966
- CN-A- 102 048 699
- LONG LEI ET AL.: 'Preparation and Quality Control of Triptorelin Acetate Microsphere.' JOURNAL OF HUBEI UNIVERSITY FOR NATIONALITIES (MEDICAL EDITION) vol. 28, no. 2, June 2011, pages 11 - 13, XP008173917

## Description

### TECHNICAL FIELD

The present invention relates to the field of pharmaceutical preparations and, particularly, to a composition of long-acting sustained release triptorelin microspheres, methods for preparing the same and use of the same.

### BACKGROUND ART

Gonadotropin-releasing hormone (GnRH), also known as luteinizing hormone-releasing hormone (LHRH), is a decapeptide hormone closely related to reproductive functions. When an exogenous LHRH or an analogue thereof is administrated with a physiological pulse frequency (once per 90 min) for a short period and at a small dose, it produces some promoting effects to the pituitary-gonadal system, and hence is used clinically for treating symptoms such as sexual dysfunction, anovulation, delayed puberty, etc. When it is administrated with a non-physiological pulse frequency for a long period and at a large dose, it can inhibit the hypophysis from secreting luteinizing hormone and follicle stimulating hormone, resulting in a decrease in the hormone secretion capacity of gonad and the atrophy of sexual organs. It is thus used clinically for treating some hormone-dependent diseases such as prostate cancer, hysteromyoma, breast carcinoma, adenomyosis, precocious puberty, etc. The LHRH and analogues thereof used clinically at the moment include triptorelin, buserelin, gonadorelin, leuprorelin, goserelin, etc.

Triptorelin is a synthetic LHRH analogue. Triptorelin modifies the structure of LHRH by substituting the sixth glycine in the natural LHRH with a D-tryptophan. Its bioactivity is 100 times of the natural LHRH, and it has significant effects in treating diseases such as prostate cancer, adenomyosis, hysteromyoma, breast carcinoma, etc.

The administration of triptorelin to its clinical indications typically requires a patient be on the medication for a long period. Thus, in order to improve patient compliance, triptorelin has been developed into long-acting sustained release preparations. Currently, the commercially available long-acting sustained release preparations of triptorelin are microsphere injections, including the products for administration once per 4 weeks, once per 12 weeks, and once per 24 weeks.

It is recognized that triptorelin must be administrated at large doses for a long period of time in order to decrease the gonadal hormone secretion capacity of the gonad and the atrophy of sexual organs, thus achieving the purpose of treating diseases such as hormone-dependent prostate cancer, etc. (Qinghua Chen et al., Development in research on microsphere drug delivery systems of polypeptide and protein drugs, Foreign Medical Sciences - Section on Pharmacy, 1997, 24(3): 129-133). Accordingly, unlike most other pharmaceutical microspheres, for which the smaller the initial release the better, an ideal long-acting microsphere preparation of triptorelin, after its injection, needs a relatively large initial release, so as to maintain the pharmaceutical effects until a later stage of drug delivery. Several LHRH analogue microspheres, which have been commercially available, are mostly of this drug release mode, for example, the microspheres of leuprorelin have an initial release up to above 20% in 1-2 days (Qinghua Chen et al., Development of research on microsphere drug delivery systems of polypeptide and protein drugs, Foreign Medical Sciences - Section on Pharmacy, 1997, 24(3): 129-133), while the triptorelin pamoate microspheres from Debiopharm S.A. have a drug release of up to above 40% in 1-2 days, during that time the concentration of testosterone in blood plasma rises in the early stage of drug delivery, reaches a maximum value on about the 4^{th} day, and then decreases to a castration level, thus producing the pharmaceutical effects thereof (American FDA documents, FDA Application No: (NDA)020715).
WO 2004/112752 discloses a method for preparing a mixed formulation of sustained release microspheres with various compositions by a continuous one-step process. This method is characterized by preparing the mixed formulation of sustained release microspheres with different compositions by a continuous one-step process by continuously introducing the mixed fluids into a dryer from the two or more different fluids for preparation of sustained release microspheres containing a biodegradable polymer, a drug, an additive and a solvent with different types of contents or both of the components, by controlling the mixing ratios of the fluids according to the time, unlike a conventional method including spray-drying a mixture of microspheres containing a biodegradable polymer, a drug, an additive and a solvent with a single composition.

However, the triptorelin microspheres prepared by a double emulsion-solvent evaporation process have a very low initial release, which makes the drug incapable of acting as soon as possible after its administration. Thus, there remains a need for developing extended release triptorelin microspheres that achieve a high initial release profile.

### SUMMARY

Described herein are embodiments directed to triptorelin microspheres incorporating glucose. As a result, the initial release of the drug *in vivo* can be increased, thus promoting the drug to act as soon as possible. The present disclosure thus provides a pharmaceutical composition of sustained release triptorelin microspheres, the triptorelin microspheres comprising triptorelin or a salt thereof, copolymers of lactide and glycolide, and glucose, wherein the content by weight of glucose is 0.1-10%, or 0.5-10%, or 0.5-5%, or 0.5-2%, or 1%; the content by weight of triptorelin or the salt thereof is 1-30%, or 2-20%, or 5-15%; the content by weight of copolymers of lactide and glycolide is 60-98.9%, or 75-97.8%, or 83-94.5%.

Specifically, in the pharmaceutical composition of sustained release triptorelin microspheres of the present invention, the content by weight of triptorelin or the salt thereof is 1-30%, the content by weight of copolymers of lactide and glycolide is 60-98.9%, the content by weight of glucose is 0.1-10%.

In the pharmaceutical composition of sustained release triptorelin microspheres of the present invention, the content by weight of triptorelin or the salt thereof is 2-20%, the content by weight of copolymers of lactide and glycolide is 70-97.5%, the content by weight of glucose is 0.5-10%.

In the pharmaceutical composition of sustained release triptorelin microspheres of the present invention, the content by weight of triptorelin or the salt thereof is 2-20%, the content by weight of copolymers of lactide and glycolide is 75-97.5%, the content by weight of glucose is 0.5-5%.

In the pharmaceutical composition of sustained release triptorelin microspheres of the present invention, the content by weight of triptorelin or the salt thereof is 5-15%, the content by weight of copolymers of lactide and glycolide is 83-94.5%, the content by weight of glucose is 0.5-2%.

In the pharmaceutical composition of sustained release triptorelin microspheres of the present invention, the content by weight of triptorelin or the salt thereof is 10%, the content by weight of copolymers of lactide and glycolide is 89%, the content by weight of glucose is 1%.

The microspheres as disclosed herein is: Small spherical or spherical-like particles consist of drug dissolved and (or) dispersed homogeneously throughout a polymer material, with a particle size ranging in 1-500µm, and generally prepared as suspensions for injection.

The copolymers of lactide and glycolide is also referred to as poly(lactide-co-glycolide), abbreviated as PLGA. The molar ratio of lactide to glycolide in said PLGA is 90:10 to 40:60, or 75:25 to 40:60, or 60:40 to 40:60, or 50:50.

The intrinsic viscosity of PLGA is 0.10-0.70 dL/g, preferably in the range of 0.15-0.50 dL/g, and optimally in the range of 0.20-0.35 dL/g. A method for measuring the intrinsic viscosity of PLGA is as follows: preparing an about 0.5% (w/v) solution of PLGA in chloroform, and determining the intrinsic viscosity of PLGA at 30°C using a Cannon-Fenske glass capillary viscometer.

The PLGA described in the present invention may have a molecular weight of 5,000-100,000 Dalton, preferably 10,000-75,000 Dalton, and more preferably 15,000-40,000 Dalton. As used herein, the term "molecular weight" refers to "weight average molecular weight."

As used herein, the molar ratio of lactide to glycolide and the intrinsic viscosity of PLGA are shown hereinafter in brackets. For example, "PLGA (75/25, 0.5, 75000)" represents poly(lactide-co-glycolide) with a molar ratio of lactide to glycolide of 75:25, an intrinsic viscosity of 0.5 dl/g and a molecular weight of 75,000 Dalton.

The drug loading amount described in the present invention is the actual drug loading amount, which is calculated as follows: drug loading amount = [amount of drug in microspheres/(amount of drug in microspheres + amount of polymers)] × 100%.

The salt of triptorelin in the sustained release microsphere provided by the present invention can be a water-soluble salt such as acetates, etc.

The sustained release triptorelin microspheres provided by the present invention is prepared by a conventional double emulsion-solvent evaporation process, wherein the glucose is added into an inner water phase, and a preferred process is as follows: PLGA is dissolved in dichloromethane to form an oil phase, triptorelin and glucose are weighted and dissolved in deionized water to form a water phase; the water phase is added into the oil phase, and then subjected to shearing emulsification so as to obtain a w/o primary emulsion. Then, the primary emulsion is added into a polyvinyl alcohol (PVA) solution, homogeneously emulsified to obtain a w/o/w double emulsion, then the organic solvent is removed therefrom, and the residue is washed and filtered to obtain the microspheres.

The present invention further provides a use of the triptorelin microspheres in preparing drugs for treating prostate cancer, sexual precocity, adenomyosis, female infertility, and hysteromyoma.

The microspheres provided by the present invention can be made into the form of sterile powder, wherein the sterile powder comprises the composition of triptorelin microspheres and mannitol, and it can be prepared as follows: rinsing the composition of triptorelin microspheres with water for injection and transferring into a freeze-drying tray, adding mannitol and a proper amount of water for injection therein, placing the freeze-drying tray in a freeze drier for freeze-drying; and subjecting the freeze-dried product to sieving and mixing, aseptic filling and capping, so as to obtain the sterile powder. Before being administrated to a patient, the sterile powder is suspended in a pharmaceutically acceptable dispersion solvent, wherein the dispersion solvent may be one or more of a suspending agent, a pH regulator, an isoosmotic adjusting agent and a surfactant, together with water for injection; the suspending agent may be one or more of sodium carboxymethyl cellulose, polyvinyl alcohol, polyvinylpyrrolidone, sodium alginate, and glycerol; the isoosmotic adjusting agent may be one or more of sodium chloride, glucose, mannitol and sorbitol; and the surfactant is a nonionic surfactant, such as polysorbate series (e.g., polysorbate 80, polysorbate 60, etc.), or triblock copolymers of poly(propylene glycol) flanked by poly(ethylene glycol), sold under the trade name of Poloxamer (e.g., Poloxamer 188, etc.).

The sustained release triptorelin microspheres provided by the present invention is used for intramuscular or subcutaneous injection, with the administration dose thereof being 3.75 mg/28days (calculated by the amount of

### BRIEF DESCRIPTION OF THE DRAWINGS

Fig. 1: a logarithmic graph of dog *in vivo* blood concentration-time curves of triptorelin microspheres in Test Example 1;
Fig. 2: a graph *of in vitro* release curves of triptorelin microspheres in Test Example 2;
Fig. 3: a graph of rat serum testosterone concentration-time curves of triptorelin microspheres in Test Example 3;
Fig. 4: a graph of *in vitro* release curves of triptorelin microspheres in Test Example 4;
Fig. 5: a graph of rat serum testosterone concentration-time curves of triptorelin microspheres in Test Example 5;
Fig. 6: a graph of *in vitro* release curves of triptorelin microspheres in Test Example 6;
Fig. 7: a graph of rat serum testosterone concentration-time curves of triptorelin microspheres in Test Example 7.

### DETAILED DESCRIPTION

The present disclosure will be further illustrated by the following examples and test examples, which will not limit the scope of the present invention in any way.

### Example 1

1.76 g of PLGA (50/50, 0.51, 75000) was weighed and dissolved in 6 ml of dichloromethane to form an oil phase, 200 mg of triptorelin acetate and 40 mg of glucose were weighed and dissolved in 0.6 ml of water to form a water phase; and then the oil phase was added into the water phase to give a mixture which was emulsified at 17500 rpm for 60 s to obtain a primary emulsion. The primary emulsion was added into 1000 ml of a 0.5% PVA solution at 6°C through an injector under homogenizing at 1500 rpm, and then it was homogeneously emulsified for 2 min to obtain a double emulsion. The double emulsion was transferred to a cantilever mixer rotating at a speed of 600 rpm, and was stirred for 5 h to volatilize and remove the organic solvent; the residue was filtered by a screen, washed by deionized water, and freeze-dried to obtain powdery microspheres. The microspheres had a drug loading amount of 9.42% and an entrapment efficiency of 94.2%.

### Example 2

1.46 g of PLGA (65/35, 0.37, 45000) was weighed and dissolved in 10 ml of dichloromethane to form an oil phase, 400 mg of triptorelin acetate and 140 mg of glucose were weighed and dissolved in 1.0 ml of water to form a water phase; and then the water phase was added into the oil phase to give a mixture which was emulsified at 15000 rpm for 60 s to obtain a primary emulsion. The primary emulsion was added into 1000 ml of a 1.0% PVA solution at 6°C through an injector under homogenizing at 1800 rpm, and then it was homogeneously emulsified for 4 min to obtain a double emulsion. The double emulsion was transferred to a cantilever mixer rotating at a speed of 600 rpm, and was stirred for 5 h to volatilize and remove the organic solvent; and the residue was filtered by a screen, washed by deionized water, and freeze-dried to obtain powdery microspheres. The microspheres had a drug loading amount of 17.82% and an entrapment efficiency of 89.1%.

### Example 3

1.698 g of PLGA (75/25, 0.50, 70000) was weighed and dissolved in 12 ml of dichloromethane to form an oil phase, 300 mg of triptorelin acetate and 2 mg of glucose were weighed and dissolved in 1.2 ml of water to form a water phase; and then the oil phase was added into the water phase to give a mixture which was emulsified at 13500 rpm for 90 s to obtain a primary emulsion. The primary emulsion was added into 1000 ml of a 2.0% PVA solution at 4°C through an injector under homogenizing at 1500 rpm, and then was homogeneously emulsified for 3 min to obtain a double emulsion. The double emulsion was transferred to a cantilever mixer rotating at a speed of 500 rpm, and was stirred for 5 h to volatilize and remove the organic solvent; and the residue was filtered by a screen, washed by deionized water, and freeze-dried to obtain powdery microspheres. The microspheres had a drug loading amount of 13.71 % and an entrapment efficiency of 91.4%.

### Example 4

1.50 g of PLGA (75/25, 0.50, 70000) was weighed and dissolved in 12 ml of dichloromethane to form an oil phase, 300 mg of triptorelin acetate and 200 mg of glucose were weighed and dissolved in 1.2 ml of water to form a water phase; and then the oil phase was added into the water phase to give a mixture which was emulsified at 17500 rpm for 90 s to obtain a primary emulsion. The primary emulsion was added into 1000 ml of a 1.0% PVA solution at 4°C through an injector under homogenizing at 2000 rpm, and then was homogeneously emulsified for 4 min to obtain a double emulsion. The double emulsion was transferred to a cantilever mixer rotating at a speed of 400 rpm, and was stirred for 5 h to volatilize and remove the organic solvent; and the residue was filtered by a screen, washed by deionized water, and freeze-dried to obtain powdery microspheres. The microspheres had a drug loading amount of 13.52% and an entrapment efficiency of 90.13%.

### Example 5

1.88 g of PLGA (50/50, 0.25, 26000) was weighed and dissolved in 10 ml of dichloromethane to form an oil phase, 100 mg of triptorelin acetate and 20 mg of glucose were weighed and dissolved in 1.0 ml of water to form a water phase; and then the oil phase was added into the water phase to give a mixture which was emulsified at 15000 rpm for 90 s to obtain a primary emulsion. The primary emulsion was added into 1000 ml of a 1.0% PVA solution at 6°C through an injector under homogenizing at 2100 rpm, and then was homogeneously emulsified for 2 min to obtain a double emulsion. The double emulsion was transferred to a cantilever mixer rotating at a speed of 600 rpm, and was stirred for 5 h to volatilize and remove the organic solvent; and the residue was filtered by a screen, washed by deionized water, and freeze-dried to obtain powdery microspheres. The microspheres had a drug loading amount of 4.59% and an entrapment efficiency of 91.8%.

### Example 6

1.80 g of PLGA (50/50, 0.25, 26000) was weighed and dissolved in 10 ml of dichloromethane to form an oil phase, 100 mg of triptorelin acetate and 100 mg of glucose were weighed and dissolved in 1.0 ml of water to form a water phase; and then the oil phase was added into the water phase to give a mixture which was emulsified at 15000 rpm for 90 s to obtain a primary emulsion. The primary emulsion was added into 1000 ml of a 1.0% PVA solution at 6°C through an injector under homogenizing at 2100 rpm, and then was homogeneously emulsified for 2 min to obtain a double emulsion. The double emulsion was transferred to a cantilever mixer rotating at a speed of 600 rpm, and was stirred for 5 h to volatilize and remove the organic solvent; and the residue was filtered by a screen, washed by deionized water, and freeze-dried to obtain powdery microspheres. The microspheres had a drug loading amount of 4.67% and an entrapment efficiency of 93.4%.

### Reference Example 7

1.90 g of PLGA (90/10, 0.42, 53000) was weighed and dissolved in 10 ml of dichloromethane to form an oil phase, 40 mg of triptorelin acetate and 60 mg mannitol were weighed and dissolved in 1.0 ml of water to form a water phase; and then the water phase was added into the oil phase to give a mixture which was emulsified at 13500 rpm for 180 s to obtain a primary emulsion. The primary emulsion was added into a reaction kettle containing 1000 ml of a 1.0% PVA solution at 10°C through an injector under homogenizing at 1800 rpm, and then was homogeneously emulsified for 3 min to obtain a double emulsion. With a rotation speed of 600 rpm, it was stirred for 5 h to volatilize and remove the organic solvent; and the residue was filtered by a screen, washed by deionized water, and freeze-dried to obtain powdery microspheres. The microspheres had a drug loading amount of 1.84% and an entrapment efficiency of 92.0%.

### Reference Example 8

1.698 g of PLGA (75/25, 0.49, 70000) was weighed and dissolved in 12 ml of dichloromethane to form an oil phase, 300 mg of triptorelin acetate and 2 mg mannitol were weighed and dissolved in 1.2 ml of water to form a water phase; and then the water phase was added into the oil phase to give a mixture which was emulsified at 15000 rpm for 180 s to obtain a primary emulsion. The primary emulsion was added into a reaction kettle containing 1000 ml of a 1.0% PVA solution at 10°C through an injector under homogenizing at 1800 rpm, and then was homogeneously emulsified for 3 min to obtain a double emulsion. With a rotation speed of 600 rpm, it was stirred for 5 h to volatilize and remove the organic solvent; and the residue was filtered by a screen, washed by deionized water, and freeze-dried to obtain powdery microspheres. The microspheres had a drug loading amount of 13.84% and an entrapment efficiency of 92.3%.

### Reference Example 9

1.88 g of PLGA (50/50, 0.25, 26000) was weighed and dissolved in 10 ml of dichloromethane to form an oil phase, 100 mg of triptorelin acetate and 20 mg mannitol were weighed and dissolved in 1.0 ml of water to form a water phase; and then the oil phase was added into the water phase to give a mixture which was emulsified at 15000 rpm for 90 s to obtain a primary emulsion. The primary emulsion was added into 1000 ml of a 1.0% PVA solution at 6°C through an injector under homogenizing at 2100 rpm, and then was homogeneously emulsified for 2 min to obtain a double emulsion. The double emulsion was transferred to a cantilever mixer rotating at a speed of 600 rpm, and was stirred for 5 h to volatilize and remove the organic solvent; and the residue was filtered by a screen, washed by deionized water, and freeze-dried to obtain powdery microspheres. The microspheres had a drug loading amount of 4.53% and an entrapment efficiency of 90.6%.

### Reference Example 10

1.80 g of PLGA (50/50, 0.25, 26000) was weighed and dissolved in 10 ml of dichloromethane to form an oil phase, 100 mg of triptorelin acetate and 100 mg mannitol were weighed and dissolved in 1.0 ml of water to form a water phase; and then the oil phase was added into the water phase to give a mixture which was emulsified at 15000 rpm for 90 s to obtain a primary emulsion. The primary emulsion was added into 1000 ml of a 1.0% PVA solution at 6°C through an injector under homogenizing at 2100 rpm, and then was homogeneously emulsified for 2 min to obtain a double emulsion. The double emulsion was transferred to a cantilever mixer rotating at a speed of 600 rpm, and was stirred for 5 h to volatilize and remove the organic solvent; and the residue was filtered by a screen, washed by deionized water, and freeze-dried to obtain powdery microspheres. The microspheres had a drug loading amount of 4.63% and an entrapment efficiency of 92.6%.

### Example 11

1.86 g of PLGA (100/0, 0.37, 50000) was weighed and dissolved in 10 ml of dichloromethane to form an oil phase, 100mg triptorelin acetate and 40 mg of glucose were weighed and dissolved in 1.0 ml of water to form a water phase; and then the water phase was added into the oil phase to give a mixture which was emulsified at 13500 rpm for 120 s to obtain a primary emulsion. The primary emulsion was added into 1000 ml of a 1.0% PVA solution at 10°C through an injector under homogenizing at 2000 rpm, and then was homogeneously emulsified for 3 min to obtain a double emulsion. The homogenized double emulsion was transferred to a cantilever mixer rotating at a speed of 600 rpm, and was stirred for 5 h to volatilize and remove the organic solvent; and the residue was filtered by a screen, washed by deionized water, and freeze-dried to obtain powdery microspheres. The microspheres had a drug loading amount of 4.74% and an entrapment efficiency of 94.8%.

### Example 12

3.64 g of PLGA (75/25, 0.68, 100000) was weighed and dissolved in 40 ml of dichloromethane to form an oil phase, 40 mg of triptorelin acetate and 320 mg of glucose were weighed and dissolved in 4.0 ml of water to form a water phase; and then the oil phase was added into the water phase to give a mixture which was emulsified at 13500 rpm for 180 s to obtain a primary emulsion. The primary emulsion was added into 4000 ml of a 1.0% PVA solution at 8°C through an injector under homogenizing at 1200 rpm, and then was homogeneously emulsified for 3 min to obtain a double emulsion. The double emulsion was transferred to a cantilever mixer rotating at a speed of 300 rpm, and was stirred for 5 h to volatilize and remove the organic solvent; and the residue was filtered by a screen, washed by deionized water, and freeze-dried to obtain powdery microspheres. The microspheres had a drug loading amount of 0.98% and an entrapment efficiency of 98.0%.

### Example 13

5.12 g of PLGA (85/15, 0.11, 7100) was weighed and dissolved in 50 ml of dichloromethane to form an oil phase, 2.4 g triptorelin acetate and 480 mg of glucose were weighed and dissolved in 5.0 ml of water to form a water phase; and then the water phase was added into the oil phase to give a mixture which was emulsified at 17500 rpm for 90 s to obtain a primary emulsion. The primary emulsion was added into 4000 ml of a 1.0% PVA solution at 8°C through an injector under homogenizing at 1300 rpm, and then was homogeneously emulsified for 3 min to obtain a double emulsion. The homogenized double emulsion was transferred to a cantilever mixer rotating at a speed of 300 rpm, and was stirred for 5 h to volatilize and remove the organic solvent; and the residue was filtered by a screen, washed by deionized water, and freeze-dried to obtain powdery microspheres. The microspheres had a drug loading amount of 26.0% and an entrapment efficiency of 86.7%.

### Comparative Example 1

1.90 g of PLGA (50/50, 0.23, 26000) and 100 mg of triptorelin acetate were weighed and dissolved respectively in 10 ml of dichloromethane, and 1.0 ml of water under stirring, so as to obtain clear solutions; the dissolved dichloromethane phase was added into the dissolved water phase to give a mixture which was emulsified at 15000 rpm for 90 s to obtain a primary emulsion. The primary emulsion was added into 1000 ml of a 1% PVA solution at 6°C through an injector under homogenizing at 2100 rpm, and then was homogeneously emulsified for 2 min to obtain a double emulsion. The double emulsion was transferred to a cantilever mixer rotating at a speed of 600 rpm, and was stirred for 5 h to volatilize and remove the organic solvent; and the residue was filtered by a screen, washed by deionized water, and freeze-dried to obtain powdery microspheres. The microspheres had a drug loading amount of 4.49% and an entrapment efficiency of 89.8%.

### Test Example 1: Dog In Vivo Pharmacokinetics Tests of Triptorelin Microspheres Comprising Glucose/Mannitol With Different Contents.

### 1) Test materials

Test drugs: triptorelin microspheres prepared according to Examples 5, 6, Reference Examples 9 and 10, which contained 1% and 5% glucose and 1% and 5% of mannitol, respectively, and a drug loading of about 4.5-4.7%.

Control group: triptorelin microspheres comprising no glucose/mannitol and a drug loading of about 4.5% as prepared according to Comparative Example 1.

Experimental animals: 20 healthy male Beagle dogs with body weights of 9-12 kg;
Test instruments: a QTRAP5500 mass spectrometer fitted with an ionspray ionization source (Applied Biosystem, Inc.);
an Agilent 1290 high performance liquid chromatography system comprising a dual infusion pump, an autosampler and a column oven;
an Anke TGL-16G Feige desk centrifuge, (ShangHai Anting Scientific Instrument Factory); and
a Turbo Vap LV pressure blowing concentrator, (Biotage, Inc).

### 2) Methods and results

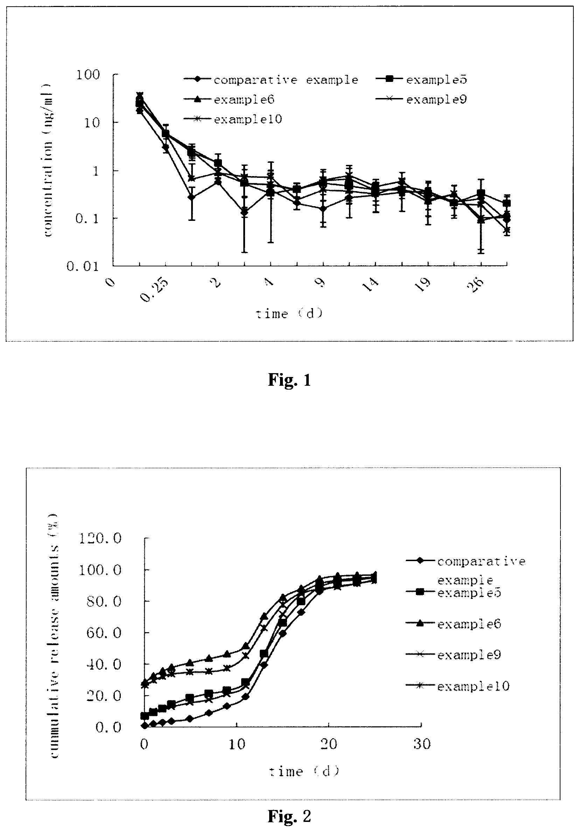

20 healthy male Beagle dogs were divided randomly into 5 groups, including a control group (Comparative Example 1), a first experimental group (Example 5), a second experimental group (Example 6), a third experimental group (Reference Example 9) and a fourth experimental group (Reference Example 10), respectively, all of which were subjected to drug administration by intramuscular injection at a dose of 0.3 mg/kg, blood samples were collected before administration and 1 h, 6 h, 1 d, 2 d, 3 d, 4 d, 6 d, 9 d, 11 d, 14 d, 16 d, 19 d, 23 d, 26 d and 30 d after administration, respectively, the concentrations of triptorelin in the blood plasma of the Beagle dogs were determined by an LC-MS/MS method, and the test results were shown in Table 1 and Fig. 1.

### LC-MS/MS method:

Liquid phase conditions: the column is Venusil MP-C18, and the mobile phase is 0.05% of acetic acid solution-methonal with the flow rate of 0.6mL/min. The column remained at 40°C, and the injection volume is 10µl.

Mass spectrometer conditions: the ionization source is an electro spray ionization (ESI) source; the source voltage is maintained at 5500V, and operated in positive mode; the scanner mode is multiple reaction monitoring (MRM), using the transitions of m/z 656.5-249.1 and m/z 656.5-110.1 for quantitative analysis; the declustering potential (DP) is 50V and the collision energy (CE) are 42eV and 90eV respectively.

The results show that the triptorelin microspheres released drugs immediately after administration, and Cₘₐₓ of the triptorelin microspheres containing glucose/mannitol (i.e., glucose or mannitol) was significantly higher than those containing no glucose/mannitol. Thus, the presence of glucose/mannitol in the triptorelin microspheres was shown to increase the *in vivo* initial release of triptorelin as compared to triptorelin microspheres of a similar drug loading but without glucose or mannitol.

**Table 1 Blood concentrations (ng/mL) at different times after the microspheres were administrated by intramuscular injection to each group of dogs**

| Time (D) | Comparative Example 1 | Example 5 | Example 6 | Reference Example 9 | Reference Example 10 |
|---|---|---|---|---|---|
| 0 | 0 | 0 | 0 | 0 | 0 |
| 0.0417 | 17.667±2.098 | 29.667±2.223 | 35.5±3.064 | 26.9±1.852 | 36.734±3.751 |
| 0.25 | 3.017±0.045 | 7.69±3.335 | 5.917±2.599 | 5.917±2.61 | 5.683±1.192 |
| 1 | 0.272±0.182 | 3.363±0.767 | 2.44±0.623 | 3.773±0.803 | 0.672±0.709 |
| 2 | 0.575±0.793 | 2.437±0.767 | 0.891±0.193 | 1.391±0.169 | 0.908±0.371 |
| 3 | 0.13±0.025 | 0.731±0.512 | 0.541±0.251 | 0.535±0.261 | 0.738±0.567 |
| 4 | 0.391±0.484 | 0.333±0.074 | 0.507±0.476 | 0.513±0.483 | 0.724±0.762 |
| 6 | 0.208±0.058 | 0.413±0.131 | 0.395±0.133 | 0.405±0.033 | 0.242±0.024 |
| 9 | 0.16±0.078 | 0.539±0.149 | 0.613±0.304 | 0.636±0.404 | 0.393±0.328 |
| 11 | 0.271±0.07 | 0.471±0.243 | 0.654±0.451 | 0.794±0.456 | 0.371±0.269 |
| 14 | 0.307±0.078 | 0.394±0.260 | 0.376±0.185 | 0.466±0.085 | 0.319±0.182 |
| 16 | 0.347±0.037 | 0.393±0.253 | 0.423±0.163 | 0.596±0.298 | 0.481±0.188 |
| 19 | 0.37±0.068 | 0.325±0.216 | 0.224±0.15 | 0.244±0.134 | 0.369t0.219 |
| 23 | 0.22±0.104 | 0.21±0.017 | 0.319±0.155 | 0.324±0.165 | 0.202±0.102 |
| 26 | 0.258±0.043 | 0.331±0.313 | 0.09±0.113 | 0.102±0.111 | 0.19±0.168 |
| 30 | 0.091±0.047 | 0.203±0.098 | 0.122±0.16 | 0.104±0.175 | 0.057±0.067 |

### Test Example 2: In Vitro Release Test of Triptorelin Microspheres comprising Glucose/Mannitol With Different Contents

### 1) Test materials

Test drugs: triptorelin microspheres prepared according to Examples 5, 6, Reference Examples 9 and 10, which contained 1% and 5% of glucose and 1% and 5% of mannitol, respectively, and a drug loading of about 4.5-4.7%.

Control group: triptorelin microspheres containing no release regulator and with a drug loading amount of about 4.5% prepared according to Comparative Example 1.

Test instruments: an Agilent 1290 high performance liquid chromatography system comprising a dual infusion pump, an autosampler and a column oven;
an Anke TGL-16G Feige desk centrifuge (ShangHai Anting Scientific Instrument Factory);

### 2) Methods and results

Methods: the microspheres weighed were placed in centrifuge tubes, a release medium (methanol: water = 5:95) was added therein and subjected to vortex treatment for 1 min. They were then put in a water bath oscillator of 37°C±0.5°C for oscillation, and the centrifuge tubes were taken out after a period of 3 h, 1 d, 2 d, 3 d, 4 d, 5 d and so on, respectively, and subjected to centrifugation with a rotation speed of 3600 rpm at 5-8°C for 15 min. The contents of triptorelin in the centrifugate were determined so as to calculate the cumulative release amounts (%), the test results were shown in Table 2 and Fig. 2.

The *in vivo* and *in vitro* research data of Test Example 1 and Test Example 2 were subjected to Boltzmann curve fitting by the Origin software, the results were shown in Table 3.

The results show that (a) the formulations with glucose/mannitol, when compared with those without glucose/mannitol, significantly increased the *in vitro* initial (0-3 hours) release amount of triptorelin; (b) the *in vitro* initial (0-3 hours) release amount of triptorelin increased with the increase of glucose/mannitol content; (c) the *in vitro* and *in vivo* release data obtained by an *in vitro* assay method had a good correlation, with all R values being above 0.9 (Table 3).

**Table 2 In Vitro cumulative release amounts (%) of triptorelin microspheres**

| Time (D) | Comparative Example 1 | Example 5 | Example 6 | Reference Example 9 | Reference Example 10 |
|---|---|---|---|---|---|
| 0.125 | 0.9 | 6.7 | 28.5 | 7.1 | 26.3 |
| 1 | 1.8 | 11.1 | 32.6 | 11.4 | 29.7 |
| 2 | 2.9 | 14.4 | 35.5 | 15.5 | 32.1 |
| 3 | 3.8 | 19.8 | 38.0 | 19.6 | 33.7 |
| 5 | 5.0 | 19.6 | 40.9 | 22.4 | 34.9 |
| 7 | 8.8 | 21.3 | 43.5 | 24.3 | 35.2 |
| 9 | 13.1 | 35.2 | 46.3 | 30.5 | 37.3 |
| 11 | 19.4 | 42.5 | 51.7 | 36.7 | 43.2 |
| 13 | 39.3 | 46.3 | 70.5 | 47.0 | 63.0 |
| 15 | 59.3 | 65.9 | 82.4 | 71.6 | 77.7 |
| 17 | 73.0 | 75.7 | 88.2 | 76.4 | 85.7 |
| 19 | 85.9 | 75.6 | 94.3 | 82.1 | 86.5 |
| 21 | 89.5 | 82.6 | 96.1 | 89.1 | 93.5 |
| 23 | 91.2 | 82.3 | 96.5 | 90.5 | 92.1 |
| 25 | 93.5 | 89.5 | 96.9 | 93.1 | 94.1 |
| 28 | 94.6 | 92.3 | 97.1 | 94.8 | 95.9 |

**Table 3 Correlation of in vivo and in vitro release data - Boltzmann fitting equation**

| | Regression equation | R |
|---|---|---|
| Comparative Example 1 | y = 0.5699x + 37.797 | 0.950 |
| Example 5 | y = 0.6915x + 36.276 | 0.940 |
| Example 6 | y = 0.6876x + 31.901 | 0.912 |
| Reference Example 9 | y = 0.6292x + 40.801 | 0.918 |
| Reference Example 10 | y = 0.6213x + 37.517 | 0.936 |

### Test Example 3: Rat In Vivo Pharmacodynamics Tests of Triptorelin Microspheres Comprising Glucose/Mannitol With Different Contents

### 1) Test materials

Test drugs: triptorelin microspheres prepared according to Examples 5, 6, Reference Examples 9 and 10, which contained 1% and 5% of glucose and 1% and 5% of mannitol, respectively, and a drug loading of about 4.5-4.7%;
Control group: triptorelin microspheres containing no release regulator and with a drug loading amount of about 4.5% prepared according to Comparative Example 1;
Negative (solvent) control group: 1% sodium carboxymethyl cellulose, 2 ml per vial;
Rat serum hormone detection kit: rat testosterone (T) ELISA kit, from R&D systems, Inc.

Experimental animals: 56 male rats with body weights of 200-250 g;
Test instruments: a high-speed refrigerated centrifuge (Beckman Coulter, Inc., Allegra X-22R);
an ELISA reader, (Molecular Devices, Inc., M5);
an analytical balance, (Mettler-Toledo Instruments, Co., Ltd., AL104);
a circular oscillator, (IKA company, IKA MS 3 Digital, Germany).

### 2) Methods and results

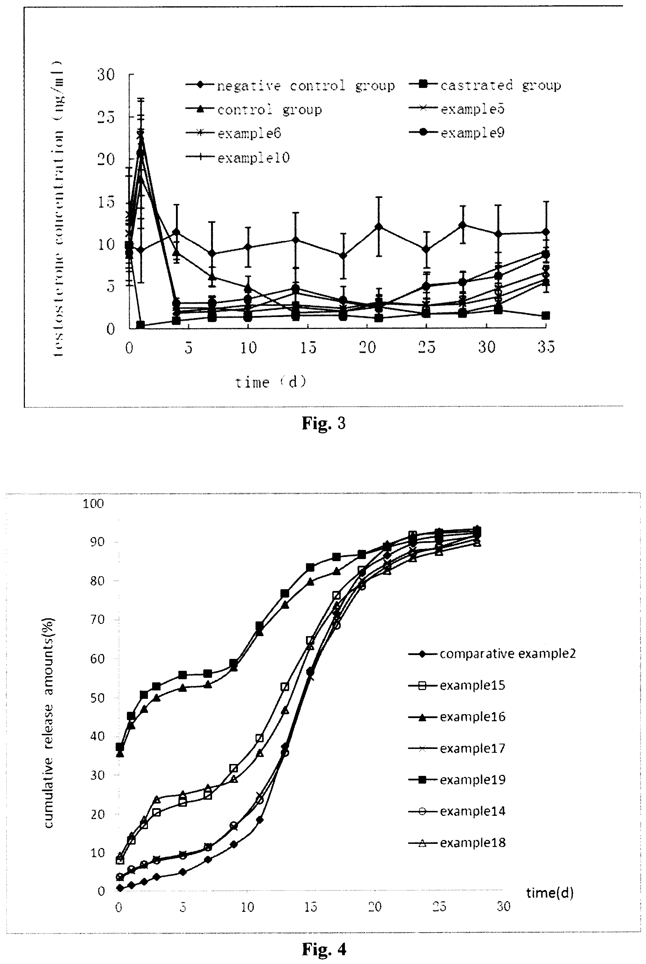

56 healthy male rats were divided randomly into 7 groups, including a castrated group, a negative control group, a control group (Comparative Example 1), a first experimental group (Example 5), a second experimental group (Example 6), a third experimental group (Reference Example 9) and a fourth experimental group (Reference Example 10), respectively, and all of which were subjected to intramuscular injection administration at a dose of 300 µg/kg, with the rats of the castrated group being castrated on the day of administration, and blood samples were collected from left and right eye sockets alternately before administration and 1 d, 4 d, 7 d, 10 d, 14 d, 18 d, 21 d, 25 d, 28 d, 32 d and 35 d after administration, respectively, with the blood sampling time at 8:00 to 10:00 in the morning. The blood samples were stood still at room temperature for 1 h, and then were centrifuged at 1000 g/min for 20 min to give supernatants, and the concentrations of serum testosterone were detected by an ELISA kit, the results were shown in Table 4 and Fig. 3.

The results show that a) the testosterone level of those groups administrated with the triptorelin microspheres without glucose or mannitol decreased to a castration level, i.e. began acting, on the 10th to 14th days; while the triptorelin microspheres containing glucose/mannitol began acting on the 4^{th} day. Therefore, the triptorelin microspheres comprising glucose/mannitol were able to act faster;
b) all the testosterone concentrations of those groups administrated with the microspheres comprising glucose were kept steady at the castration level from the 4^{th} day to the 28^{th} day; while the testosterone concentrations of those groups administrated with the microspheres comprising mannitol were substantially kept at the castration level from the 4^{th} day to the 21^{th} day, but fluctuated around the 14^{th} day. Therefore, compared to mannitol, glucose was able to maintain the pharmaceutical effects for a longer period, with more steady pharmaceutical effects over the period of release.

Table 4 Serum testosterone concentrations (ng/mL) at different times after the microspheres administrated by intramuscular injection to rats

| Time (D) | Negative control group | Castrated group | Control group | Example 5 | Example 6 | Reference Example 9 | Reference Example 10 |
|---|---|---|---|---|---|---|---|
| 0 | 9.76±2.61 | 9.78±3.05 | 8.62±3.57 | 11.23±3.56 | 13.42±4.56 | 8.87±3.84 | 12.32±6.68 |
| 1 | 9.2±3.81 | 0.38±0.14 | 17.65±5.86 | 20.46±4.73 | 22.77±4.06 | 20.67±6.46 | 23.26±1.45 |
| 4 | 11.36±3.29 | 0.92±0.15 | 8.96±1.24 | 2.44±0.8 | 2.04±0.66 | 2.97±0.57 | 1.82±0.49 |
| 7 | 8.77±3.81 | 1.34±0.5 | 6.06±1.16 | 2.46±1.2 | 2.33±1.50 | 3.01±0.69 | 2.02±0.67 |
| 10 | 9.52±2.34 | 1.39±0.39 | 4.83±1.34 | 2.04±1.22 | 2.74±1.52 | 3.47±0.82 | 2.38±1.12 |
| 14 | 10.38±3.28 | 1.55±0.62 | 1.84±0.7 | 2.52±0.54 | 2.72±1.66 | 4.73±2.27 | 4.19±1.22 |
| 18 | 8.51±2.67 | 1.49±0.42 | 1.98±0.38 | 2.01±1.03 | 2.31±1.23 | 3.25±1.61 | 3.06±1.80 |
| 21 | 11.95±3.48 | 1.18±0.08 | 2.5±0.58 | 2.91±0.84 | 3.08±1.61 | 2.61±1.29 | 2.39±1.14 |
| 25 | 9.2±2.13 | 1.72±0.34 | 1.72±0.46 | 2.7±0.75 | 2.69±1.44 | 4.86±1.43 | 5.03±1.52 |
| 28 | 12.14±2.25 | 1.65±0.3 | 1.87±0.34 | 2.84±0.91 | 3.17±1.94 | 5.37±1.20 | 5.43±1.18 |
| 32 | 11.05±3.4 | 2.07±0.38 | 2.78±0.35 | 3.69±1.15 | 4.63±1.12 | 6.08±1.23 | 7.06±1.81 |
| 35 | 11.25±3.64 | 1.39±0.17 | 5.37±0.25 | 5.73±1.55 | 6.58±0.53 | 8.54±0.86 | 8.98±1.38 |

### Example 14

1.798 g of PLGA (50/50, 0.25, 26000) was weighed and dissolved in 8 ml of dichloromethane to form an oil phase, 200 mg of triptorelin acetate and 2 mg of glucose were weighed and dissolved in 0.8 ml of water to form a water phase; and then the water phase was added into the oil phase to give a mixture which was emulsified at 15000 rpm for 90 s to obtain a primary emulsion. The primary emulsion was added into 1000 ml of a 1.0% PVA solution at 8°C through an injector under homogenizing at 2000 rpm, and then it was homogeneously emulsified for 3 min to obtain a double emulsion. The homogenized double emulsion was transferred to a cantilever mixer rotating at a speed of 500 rpm, and was stirred for 5 h to volatilize and remove the organic solvent; the residue was filtered by a screen, washed by deionized water, and freeze-dried to obtain powdery microspheres. The microspheres had a drug loading amount of 9.13% and an entrapment efficiency of 91.3%.

### Example 15

1.78 g of PLGA (50/50, 0.25, 26000) was weighed and dissolved in 8 ml of dichloromethane to form an oil phase, 200 mg of triptorelin acetate and 20 mg of glucose were weighed and dissolved in 0.8 ml of water to form a water phase; and then the water phase was added into the oil phase to give a mixture which was emulsified at 13500 rpm for 90 s to obtain a primary emulsion. The primary emulsion was added into 1000 ml of a 1.0% PVA solution at 8°C through an injector under homogenizing at 2000 rpm, and then it was homogeneously emulsified for 3 min to obtain a double emulsion. The homogenized double emulsion was transferred to a cantilever mixer rotating at a speed of 500 rpm, and was stirred for 5 h to volatilize and remove the organic solvent; the residue was filtered by a screen, washed by deionized water, and freeze-dried to obtain powdery microspheres. The microspheres had a drug loading amount of 9.01% and an entrapment efficiency of 90.1%.

### Example 16

1.60 g of PLGA (50/50, 0.25, 26000) was weighed and dissolved in 10 ml of dichloromethane to form an oil phase, 200 mg of triptorelin acetate and 200 mg of glucose were weighed and dissolved in 1.0 ml of water to form a water phase; and then the water phase was added into the oil phase to give a mixture which was emulsified at 17500 rpm for 90 s to obtain a primary emulsion. The primary emulsion was added into 1000 ml of a 1.0% PVA solution at 8°C through an injector under homogenizing at 1800 rpm, and then it was homogeneously emulsified for 3 min to obtain a double emulsion. The homogenized double emulsion was transferred to a cantilever mixer rotating at a speed of 300 rpm, and was stirred for 5 h to volatilize and remove the organic solvent; the residue was filtered by a screen, washed by deionized water, and freeze-dried to obtain powdery microspheres. The microspheres had a drug loading amount of 9.24% and an entrapment efficiency of 92.4%.

### Reference Example 17

1.798 g of PLGA (50/50, 0.25, 26000) was weighed and dissolved in 8 ml of dichloromethane to form an oil phase, 200 mg of triptorelin acetate and 2 mg of mannitol were weighed and dissolved in 0.8 ml of water to form a water phase; and then the water phase was added into the oil phase to give a mixture which was emulsified at 15000 rpm for 90 s to obtain a primary emulsion. The primary emulsion was added into 1000 ml of a 1.0% PVA solution at 8°C through an injector under homogenizing at 2000 rpm, and then it was homogeneously emulsified for 3 min to obtain a double emulsion. The homogenized double emulsion was transferred to a cantilever mixer rotating at a speed of 500 rpm, and was stirred for 5 h to volatilize and remove the organic solvent; the residue was filtered by a screen, washed by deionized water, and freeze-dried to obtain powdery microspheres. The microspheres had a drug loading amount of 9.12% and an entrapment efficiency of 91.2%.

### Reference Example 18

1.78 g of PLGA (50/50, 0.25, 26000) was weighed and dissolved in 8 ml of dichloromethane to form an oil phase, 200 mg of triptorelin acetate and 20 mg of mannitol were weighed and dissolved in 0.8 ml of water to form a water phase; and then the water phase was added into the oil phase to give a mixture which was emulsified at 13500 rpm for 90 s to obtain a primary emulsion. The primary emulsion was added into 1000 ml of a 1.0% PVA solution at 8°C through an injector under homogenizing at 2000 rpm, and then it was homogeneously emulsified for 3 min to obtain a double emulsion. The homogenized double emulsion was transferred to a cantilever mixer rotating at a speed of 500 rpm, and was stirred for 5 h to volatilize and remove the organic solvent; the residue was filtered by a screen, washed by deionized water, and freeze-dried to obtain powdery microspheres. The microspheres had a drug loading amount of 8.95% and an entrapment efficiency of 89.5%.

### Reference Example 19

1.60 g of PLGA (50/50, 0.25, 26000) was weighed and dissolved in 10 ml of dichloromethane to form an oil phase, 200 mg of triptorelin acetate and 200 mg of mannitol were weighed and dissolved in 1.0 ml of water to form a water phase; and then the water phase was added into the oil phase to give a mixture which was emulsified at 17500 rpm for 90 s to obtain a primary emulsion. The primary emulsion was added into 1000 ml of a 1.0% PVA solution at 8°C through an injector under homogenizing at 1800 rpm, and then it was homogeneously emulsified for 3 min to obtain a double emulsion. The homogenized double emulsion was transferred to a cantilever mixer rotating at a speed of 400 rpm, and was stirred for 5 h to volatilize and remove the organic solvent; the residue was filtered by a screen, washed by deionized water, and freeze-dried to obtain powdery microspheres. The microspheres had a drug loading amount of 8.98% and an entrapment efficiency of 89.8%.

### Example 20

1.79 g of PLGA (50/50, 0.25, 26000) was weighed and dissolved in 10 ml of dichloromethane to form an oil phase, 200 mg of triptorelin acetate and 10 mg of glucose were weighed and dissolved in 5.0 ml of water to form a water phase; and then the water phase was added into the oil phase to give a mixture which was emulsified at 15000 rpm for 90 s to obtain a primary emulsion. The primary emulsion was added into 1000 ml of a 1.0% PVA solution at 8°C through an injector under homogenizing at 2200 rpm, and then it was homogeneously emulsified for 3 min to obtain a double emulsion. The homogenized double emulsion was transferred to a cantilever mixer rotating at a speed of 400 rpm, and was stirred for 5 h to volatilize and remove the organic solvent; the residue was filtered by a screen, washed by deionized water, and freeze-dried to obtain powdery microspheres. The microspheres had a drug loading amount of 9.22% and an entrapment efficiency of 92.2%.

### Reference Example 21

1.79 g of PLGA (50/50, 0.25, 26000) was weighed and dissolved in 10 ml of dichloromethane to form an oil phase, 200 mg of triptorelin acetate and 10 mg of mannitol were weighed and dissolved in 1.0 ml of water to form a water phase; and then the water phase was added into the oil phase to give a mixture which was emulsified at 15000 rpm for 90 s to obtain a primary emulsion. The primary emulsion was added into 1000 ml of a 1.0% PVA solution at 8°C through an injector under homogenizing at 2200 rpm, and then it was homogeneously emulsified for 3 min to obtain a double emulsion. The homogenized double emulsion was transferred to a cantilever mixer rotating at a speed of 400 rpm, and was stirred for 5 h to volatilize and remove the organic solvent; the residue was filtered by a screen, washed by deionized water, and freeze-dried to obtain powdery microspheres. The microspheres had a drug loading amount of 9.15% and an entrapment efficiency of 91.5%.

### Comparative Example 2

1.80 g of PLGA (50/50, 0.25, 26000) was weighed and dissolved in 10 ml of dichloromethane to form an oil phase, 200 mg of triptorelin acetate was weighed and dissolved in 1.0 ml of water to form a water phase; and then the water phase was added into the oil phase to give a mixture which was emulsified at 15000 rpm for 90 s to obtain a primary emulsion. The primary emulsion was added into 1000 ml of a 1% PVA solution at 8°C through an injector under homogenizing at 2000 rpm, and then was homogeneously emulsified for 3 min to obtain a double emulsion. The homogenized double emulsion was transferred to a cantilever mixer rotating at a speed of 500 rpm, and was stirred for 5 h to volatilize and remove the organic solvent; and the residue was filtered by a screen, washed by deionized water, and freeze-dried to obtain powdery microspheres. The microspheres had a drug loading amount of 9.13% and an entrapment efficiency of 91.3%.

### Test Example 4: In Vitro Release Test of Triptorelin Microspheres comprising Glucose/Mannitol With Different Contents

Test method: the same as that in Test Example 2.

### Test materials:

Test drugs: triptorelin microspheres prepared according to Example 14-16 and Reference Example 17-19.

Control group: triptorelin microspheres without glucose/mannitol as prepared according to Comparative Example 2.

The test results were shown in Table 5 and Fig. 4.

**Table 5 In Vitro cumulative release amounts (%) of triptorelin microspheres**

| Time (D) | Comparative example 2 | Example 14 | Example 15 | Example 16 | Reference Example 17 | Reference Example 18 | Reference Example 19 |
|---|---|---|---|---|---|---|---|
| 0.125 | 0.7 | 3.7 | 7.9 | 35.6 | 3.5 | 9.2 | 37.2 |
| 1 | 1.5 | 5.6 | 13.2 | 42.9 | 5.2 | 14.5 | 45.4 |
| 2 | 2.4 | 6.8 | 17.2 | 47.2 | 6.7 | 18.6 | 50.8 |
| 3 | 3.6 | 7.9 | 20.3 | 50.1 | 8.3 | 23.8 | 52.9 |
| 5 | 4.9 | 9.2 | 22.8 | 52.6 | 9.6 | 25.1 | 55.7 |
| 7 | 8.2 | 11.3 | 24.7 | 53.4 | 11.5 | 26.7 | 56.2 |
| 9 | 12.1 | 16.9 | 31.6 | 57.8 | 16.6 | 28.9 | 58.8 |
| 11 | 18.4 | 23.4 | 39.4 | 66.7 | 24.7 | 35.6 | 68.3 |
| 13 | 37.3 | 35.7 | 52.7 | 73.8 | 36.8 | 46.8 | 76.6 |
| 15 | 56.8 | 56.4 | 64.6 | 79.6 | 55.2 | 63.2 | 83.2 |
| 17 | 71.4 | 68.3 | 76.1 | 82.3 | 69.6 | 73.5 | 85.9 |
| 19 | 81.9 | 78.5 | 82.6 | 86.5 | 79.8 | 79.3 | 86.7 |
| 21 | 86.3 | 83.6 | 88.5 | 89.2 | 84.3 | 82.4 | 88.5 |
| 23 | 89.4 | 86.8 | 91.5 | 91.3 | 87.5 | 85.6 | 90.2 |
| 25 | 90 | 88.5 | 92.1 | 92.5 | 88.2 | 87.3 | 91.3 |
| 28 | 91.3 | 91.7 | 92.6 | 93.2 | 90.6 | 89.5 | 92.1 |

The results show that (a) the formulations with glucose/mannitol, when compared with those without glucose/mannitol, significantly increased the in vitro initial (0-3 hours) release amount of triptorelin; (b) the in vitro initial (0-3 hours) release amount of the microspheres increased with the increase of the glucose/mannitol content.

### Test Example 5: Rat In Vivo Pharmacodynamics Tests of Triptorelin Microspheres Comprising Glucose/Mannitol With Different Contents.

Test method: the same as that in Test Example 3.

Test drugs: triptorelin microspheres prepared according to Example 15-16, 20 and Reference Example 18-19, 21.

Control group: triptorelin microspheres without glucose/mannitol as prepared according to Comparative Example 2.

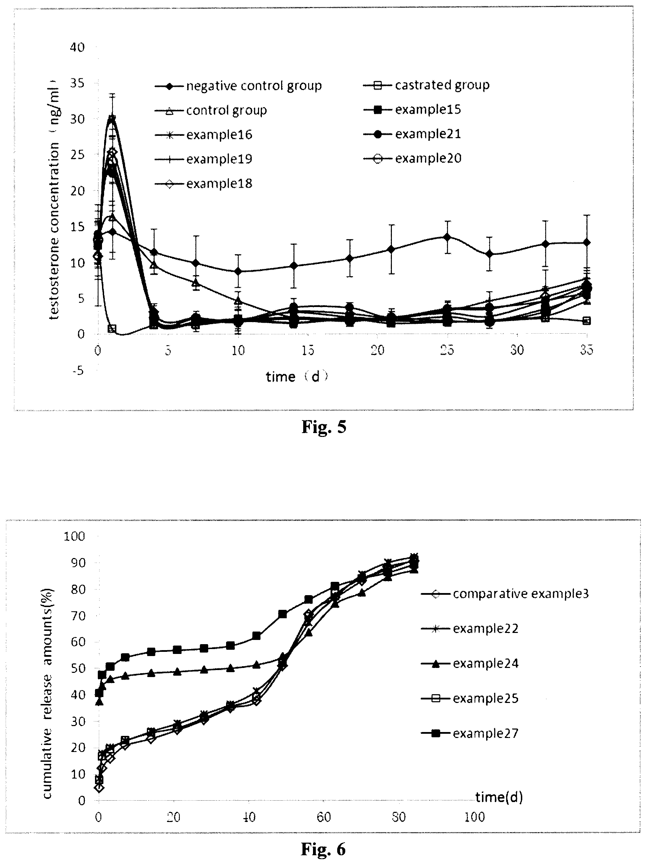

The test results were shown in Table 6 and Fig. 5.

**Table 6 Serum testosterone concentrations (ng/mL) at different time after the microspheres administrated by intramuscular injection to rats**

| Time (D) | Negative control group | Castrated group | Control group | Example 20 | Example 15 | Example 16 | Reference Example 21 | Reference Example 18 | Reference Example 19 |
|---|---|---|---|---|---|---|---|---|---|
| 0 | 13.61±2.54 | 12.56±3.16 | 13.76±3.42 | 13.22±2.61 | 12.32±3.13 | 11.66±3.56 | 14.02±4.04 | 10.86±3.17 | 9.78±5.86 |
| 1 | 14.25±3.73 | 0.72±0.17 | 16.33±4.86 | 24.07±3.15 | 23.06±4.56 | 29.71±3.76 | 18.34±5.16 | 25.31±3.26 | 30.35±2.67 |
| 4 | 11.48±3.19 | 1.22±0.12 | 9.71±1.31 | 2.36±0.82 | 2.31±0.68 | 2.24±0.46 | 2.88±1.42 | 3.05±0.89 | 2.03±1.05 |
| 7 | 9.92±3.73 | 1.31±0.53 | 7.13±1.06 | 2.23±0.33 | 1.76±1.02 | 1.59±1.25 | 2.36±0.78 | 2.16±0.53 | 1.81±0.68 |
| 10 | 8.76±2.31 | 1.86±0.36 | 4.62±1.31 | 1.86±0.27 | 2.14±1.12 | 1.97±1.53 | 1.92±1.82 | 1.67±0.41 | 2.06±1.32 |
| 14 | 9.48±3.06 | 1.53±0.59 | 2.31±0.72 | 2.14±0.56 | 1.62±0.73 | 2.35±0.96 | 3.73±1.21 | 3.16±0.88 | 3.03±1.10 |
| 18 | 10.52±2.55 | 2.07±0.43 | 1.98±0.58 | 1.79±0.49 | 2.31±1.13 | 2.01±0.43 | 3.65±0.66 | 2.82±1.03 | 2.36±1.32 |
| 21 | 11.75±3.42 | 1.48±0.18 | 1.85±0.33 | 2.21±0.17 | 1.88±0.54 | 2.32±0.81 | 2.31±1.21 | 2.35±0.74 | 2.17±0.88 |
| 25 | 13.42±2.23 | 1.66±0.26 | 1.67±0.66 | 1.82±0.42 | 2.37±1.25 | 2.87±1.14 | 3.52±0.83 | 3.37±0.61 | 3.23±1.32 |
| 28 | 11.07±2.36 | 1.73±0.31 | 1.89±0.27 | 1.73±0.38 | 1.79±0.61 | 2.42±1.67 | 3.65±1.02 | 3.45±0.55 | 4.56±1.21 |
| 32 | 12.45±3.14 | 2.12±0.29 | 2.46±0.45 | 3.08±0.25 | 3.46±1.05 | 4.54±1.32 | 4.58±1.43 | 5.16±1.32 | 6.17±2.69 |
| 35 | 12.58±3.81 | 1.72±0.16 | 4.54±0.36 | 4.25±0.72 | 5.53±1.26 | 5.63±0.83 | 6.60±0.98 | 6.82±1.48 | 7.66±1.49 |

The results show that a) the testosterone level of those groups administrated with the triptorelin microspheres without glucose or mannitol decreased to a castration level, i.e. began acting, on the 10th to 14th days; while the triptorelin microspheres containing glucose/mannitol began acting on the 4th day. Therefore, the triptorelin microspheres comprising glucose/mannitol were able to act faster;
b) all the testosterone concentrations of those groups administrated with the microspheres comprising glucose were kept steady at the castration level from the 4th day to the 28th day; while the testosterone concentrations of those groups administrated with the microspheres comprising mannitol were substantially kept at the castration level from the 4th day to the 21th day, but fluctuated around the 14th day. Therefore, as compared to mannitol, glucose was able to maintain a longer period and more steady pharmaceutical effects.

### Example 22

5.592 g of PLGA (75/25, 0.51, 70000) was weighed and dissolved in 40 ml of dichloromethane to form an oil phase, 2.4 g of triptorelin acetate and 8 mg of glucose were weighed and dissolved in 4.0 ml of water to form a water phase; and then the water phase was added into the oil phase to give a mixture which was emulsified at 13500 rpm for 90 s to obtain a primary emulsion. The primary emulsion was added into 4000 ml of a 1.0% PVA solution at 8°C through an injector under homogenizing at 1300 rpm, and then it was homogeneously emulsified for 3 min to obtain a double emulsion. The homogenized double emulsion was transferred to a cantilever mixer rotating at a speed of 350 rpm, and was stirred for 5 h to volatilize and remove the organic solvent; the residue was filtered by a screen, washed by deionized water, and freeze-dried to obtain powdery microspheres. The microspheres had a drug loading amount of 26.37% and an entrapment efficiency of 87.9%.

### Example 23

5.56 g of PLGA (75/25, 0.51, 70000) was weighed and dissolved in 50 ml of dichloromethane to form an oil phase, 2.4 g of triptorelin acetate and 40 mg of glucose were weighed and dissolved in 5.0 ml of water to form a water phase; and then the water phase was added into the oil phase to give a mixture which was emulsified at 13500 rpm for 90 s to obtain a primary emulsion. The primary emulsion was added into 4000 ml of a 1.0% PVA solution at 8°C through an injector under homogenizing at 1300 rpm, and then it was homogeneously emulsified for 3 min to obtain a double emulsion. The homogenized double emulsion was transferred to a cantilever mixer rotating at a speed of 350 rpm, and was stirred for 5 h to volatilize and remove the organic solvent; the residue was filtered by a screen, washed by deionized water, and freeze-dried to obtain powdery microspheres. The microspheres had a drug loading amount of 26.46% and an entrapment efficiency of 88.2%.

### Example 24

4.8 g of PLGA (75/25, 0.51, 70000) was weighed and dissolved in 50 ml of dichloromethane to form an oil phase, 2.4 g of triptorelin acetate and 800 mg of glucose were weighed and dissolved in 5.0 ml of water to form a water phase; and then the water phase was added into the oil phase to give a mixture which was emulsified at 13500 rpm for 90 s to obtain a primary emulsion. The primary emulsion was added into 4000 ml of a 1.0% PVA solution at 8°C through an injector under homogenizing at 1300 rpm, and then it was homogeneously emulsified for 3 min to obtain a double emulsion. The homogenized double emulsion was transferred to a cantilever mixer rotating at a speed of 350 rpm, and was stirred for 5 h to volatilize and remove the organic solvent; the residue was filtered by a screen, washed by deionized water, and freeze-dried to obtain powdery microspheres. The microspheres had a drug loading amount of 27.42% and an entrapment efficiency of 91.4%.

### Reference Example 25

5.592 g of PLGA (75/25, 0.51, 70000) was weighed and dissolved in 40 ml of dichloromethane to form an oil phase, 2.4 g of triptorelin acetate and 8 mg of mannitol were weighed and dissolved in 4.0 ml of water to form a water phase; and then the water phase was added into the oil phase to give a mixture which was emulsified at 13500 rpm for 90 s to obtain a primary emulsion. The primary emulsion was added into 4000 ml of a 1.0% PVA solution at 8°C through an injector under homogenizing at 1300 rpm, and then it was homogeneously emulsified for 3 min to obtain a double emulsion. The homogenized double emulsion was transferred to a cantilever mixer rotating at a speed of 350 rpm, and was stirred for 5 h to volatilize and remove the organic solvent; the residue was filtered by a screen, washed by deionized water, and freeze-dried to obtain powdery microspheres. The microspheres had a drug loading amount of 26.82% and an entrapment efficiency of 89.4%.

### Reference Example 26

5.56 g of PLGA (75/25, 0.51, 70000) was weighed and dissolved in 50 ml of dichloromethane to form an oil phase, 2.4 g of triptorelin acetate and 40 mg of mannitol were weighed and dissolved in 5.0 ml of water to form a water phase; and then the water phase was added into the oil phase to give a mixture which was emulsified at 12000 rpm for 90 s to obtain a primary emulsion. The primary emulsion was added into 4000 ml of a 1.0% PVA solution at 8°C through an injector under homogenizing at 1500 rpm, and then it was homogeneously emulsified for 3 min to obtain a double emulsion. The homogenized double emulsion was transferred to a cantilever mixer rotating at a speed of 300 rpm, and was stirred for 5 h to volatilize and remove the organic solvent; the residue was filtered by a screen, washed by deionized water, and freeze-dried to obtain powdery microspheres. The microspheres had a drug loading amount of 27.15% and an entrapment efficiency of 90.5%.

### Reference Example 27

4.8 g of PLGA (75/25, 0.51, 70000) was weighed and dissolved in 50 ml of dichloromethane to form an oil phase, 2.4 g of triptorelin acetate and 800 mg of mannitol were weighed and dissolved in 5.0 ml of water to form a water phase; and then the water phase was added into the oil phase to give a mixture which was emulsified at 12000 rpm for 90 s to obtain a primary emulsion. The primary emulsion was added into 4000 ml of a 1.0% PVA solution at 8°C through an injector under homogenizing at 1500 rpm, and then it was homogeneously emulsified for 3 min to obtain a double emulsion. The homogenized double emulsion was transferred to a cantilever mixer rotating at a speed of 300 rpm, and was stirred for 5 h to volatilize and remove the organic solvent; the residue was filtered by a screen, washed by deionized water, and freeze-dried to obtain powdery microspheres. The microspheres had a drug loading amount of 27.84% and an entrapment efficiency of 92.8%.

### Comparative Example 3

5.6 g of PLGA (75/25, 0.51, 70000) was weighed and dissolved in 50 ml of dichloromethane to form an oil phase, 2.4 mg of triptorelin acetate was weighed and dissolved in 5.0 ml of water to form a water phase; and then the water phase was added into the oil phase to give a mixture which was emulsified at 13500 rpm for 90 s to obtain a primary emulsion. The primary emulsion was added into 4000 ml of a 1% PVA solution at 8°C through an injector under homogenizing at 1300 rpm, and then was homogeneously emulsified for 3 min to obtain a double emulsion. The homogenized double emulsion was transferred to a cantilever mixer rotating at a speed of 350 rpm, and was stirred for 5 h to volatilize and remove the organic solvent; and the residue was filtered by a screen, washed by deionized water, and freeze-dried to obtain powdery microspheres. The microspheres had a drug loading amount of 27.5% and an entrapment efficiency of 91.7%.

### Test Example 6: In Vitro Release Test of Triptorelin Microspheres comprising Glucose/Mannitol With Different Contents

Test method: the same as that in Test Example 2.

### Test materials:

Test drugs: triptorelin microspheres prepared according to Example 22, 24 and Reference Example 25, 27.

Control group: triptorelin microspheres without glucose/mannitol prepared according to Comparative Example 3.

The test results were shown in Table 7 and Fig. 6.

**Table 7 In Vitro cumulative release amounts (%) of triptorelin microspheres**

| Time (D) | Comparative example 3 | Example 22 | Example 24 | Reference Example 25 | Reference Example 27 |
|---|---|---|---|---|---|
| 0.125 | 4.8 | 8.2 | 37.4 | 7.9 | 40.6 |
| 1 | 12.4 | 17.9 | 43.2 | 16.7 | 47.4 |
| 3 | 16.1 | 20.3 | 45.8 | 19.5 | 50.6 |
| 7 | 20.9 | 22.6 | 47.2 | 22.8 | 54.1 |
| 14 | 23.5 | 26.3 | 48.3 | 25.7 | 56.3 |
| 21 | 26.8 | 29.2 | 48.8 | 27.6 | 56.9 |
| 28 | 30.5 | 32.7 | 49.5 | 31.2 | 57.5 |
| 35 | 35.1 | 36.4 | 50.1 | 35.6 | 58.6 |
| 42 | 37.6 | 41.5 | 51.4 | 39.2 | 62.2 |
| 49 | 50.8 | 51.6 | 54.5 | 52.3 | 70.5 |
| 56 | 70.4 | 67.3 | 63.7 | 69.6 | 76.1 |
| 63 | 76.9 | 77.2 | 74.5 | 78.4 | 81.2 |
| 70 | 83.2 | 85.6 | 78.8 | 84.1 | 84.1 |
| 77 | 88.5 | 90.1 | 84.6 | 87.5 | 86.3 |
| 84 | 90.7 | 92.3 | 87.3 | 91.2 | 89.4 |

The results show that (a) the formulations with glucose/mannitol, when compared with those without glucose/mannitol, significantly increased the in vitro initial (0-3 hours) release amount of triptorelin; (b) the in vitro initial (0-3 hours) release amount of triptorelin increased with the increase of the glucose/mannitol content.

### Test Example 7: Rat In Vivo Pharmacodynamics Tests of Triptorelin Microspheres Comprising Glucose/Mannitol With Different Contents.

Test method: the same as that in Test Example 3.

Test drugs: triptorelin microspheres prepared according to Example 23-24, Reference Example 26-27.

Control group: triptorelin microspheres without glucose/mannitol prepared according to Comparative Example 3.

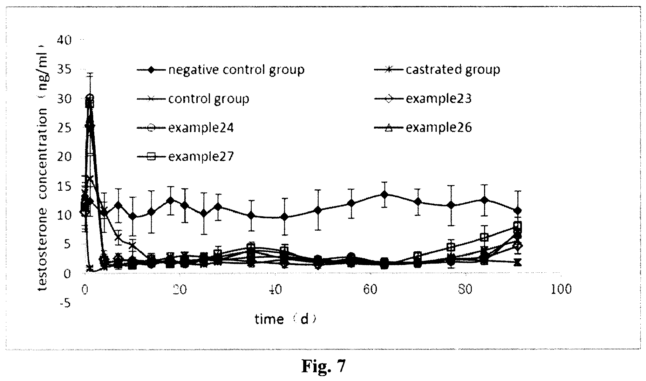

The test results were shown in Table 8 and Fig. 7.

**Table 8 Serum testosterone concentrations (ng/mL) at different time after the microspheres administrated by intramuscular injection to rats**

| Time (D) | Negative control group | Castrated group | Control group | Example 23 | Example 24 | Reference Example 26 | Reference Example 27 |
|---|---|---|---|---|---|---|---|
| 0 | 11.52±3.25 | 13.79±3.05 | 12.73±2.83 | 10.56±3.41 | 11.92±1.22 | 13.24±3.45 | 11,33±3.68 |
| 1 | 12.37±2.52 | 0.88±0.21 | 16.24±3.96 | 25.31±4.78 | 30.11±4.31 | 26.45±2.78 | 29.08±4.74 |
| 4 | 10.36±3.41 | 1.02±0.14 | 10.80±1.42 | 2.35±0.68 | 2.33±0.65 | 2.76±1.06 | 2.25±0.96 |
| 7 | 11.60±2.86 | 1.51±0.38 | 6.13±1.28 | 2.48±0.85 | 1.65±0.88 | 2.23±0.75 | 1.57±0.42 |
| 10 | 9.76±3.31 | 1.84±0.52 | 4.74±1.05 | 2.14±0.63 | 1.76±0.63 | 2.32±1.42 | 1.36±0.37 |
| 14 | 10.48±3.62 | 1.72±0.46 | 2.45±0.68 | 1.73±0.56 | 1.53±0.42 | 2.05±0.95 | 2.43±0.95 |
| 18 | 12.44±2.41 | 2.31±0.37 | 2.76±0.72 | 2.21±0.97 | 2.32±0.56 | 1.87±0.61 | 1.58±0.26 |
| 21 | 11.63±2.88 | 1.69±0.20 | 3.07±0.56 | 1.88±0.41 | 1.57±0.37 | 2.02±0.81 | 2.22±0.78 |
| 25 | 10.24±3.55 | 1.56±0.19 | 2.86±0.43 | 2.37±1.05 | 2.34±0.74 | 2.45±0.46 | 2.43±1.02 |
| 28 | 11.31±2.27 | 1.89±0.37 | 2.89±0.39 | 2.52±0.41 | 2.29±0.62 | 2.33±0.74 | 3.26±1.27 |
| 35 | 9.88±2.62 | 1.76±0.42 | 3.62±1.25 | 2.04±1.02 | 2.76±1.13 | 3.81±1.12 | 4.35±0.96 |
| 42 | 9.64±3.17 | 2.36±0.55 | 2.55±0.66 | 1.65±0.66 | 2.85±0.55 | 3,39±1.47 | 3.72±1.18 |
| 49 | 10.81±3.44 | 2.27±0.36 | 1.88±0.35 | 1.46±0.58 | 2.41±0.47 | 2.34±0.58 | 1.88±0.47 |
| 56 | 11.92±2.52 | 1.68±0.09 | 1.90±0.40 | 1.76±0.73 | 2.78±0.36 | 2.08±0.64 | 2.46±0.56 |
| 63 | 13.44±2.18 | 1.45±0.22 | 1.76±0.52 | 1.57±0.88 | 1.75±0.82 | 1.96±0.72 | 1.63±0.85 |
| 70 | 12.17±2.22 | 1.73±0.27 | 1.89±0.07 | 1.79±0.52 | 1.66±0.68 | 1.83±0.66 | 2.94±0.42 |
| 77 | 11.55±3.51 | 2.32±0.55 | 2.66±0.35 | 2.36±0.71 | 1.94±1.12 | 2.46±0.71 | 4.38±1.31 |
| 84 | 12.46±2.72 | 2.15±0.31 | 3.88±0.62 | 2.78±0.44 | 2.32±0.93 | 2.93±1.20 | 6.02±2.06 |
| 91 | 10.63±3.37 | 1.86±0.61 | 5.54±0.73 | 4.56±1.35 | 7.02±1.86 | 6.77±0.86 | 8.03±1.54 |

The results show that a) the testosterone level of those groups administrated with the triptorelin microspheres without glucose or mannitol was decreased to a castration level, i.e. began acting, on the 10th to 14th days, while the triptorelin microspheres containing glucose/mannitol began acting on the 4th day. Therefore, the triptorelin microspheres comprising glucose/mannitol were able to act faster;
b) all the testosterone concentrations of those groups administrated with the microspheres comprising glucose were kept steady at the castration level from the 4th day to the 84th day, while the testosterone concentrations of those groups administrated with the microspheres comprising mannitol were substantially kept at the castration level from the 4th day to the 70th day, but fluctuated around the 35th day, therefore, compared to mannitol, glucose was able to maintain a longer period and more steady pharmaceutical effects.

## Claims

1. A pharmaceutical composition of sustained release triptorelin microspheres, the triptorelin microspheres comprising triptorelin or a salt thereof, poly(lactide-co-glycolide), and glucose.

2. The pharmaceutical composition according to claim 1, wherein the content by weight of glucose in the triptorelin microspheres is 0.1-10%, or 0.5-10%, or 0.5-5%, or 0.5-2%, or 1%.

3. The pharmaceutical composition according to claim 2, wherein the content by weight of triptorelin or the salt thereof in the triptorelin microspheres is 1-30%.

4. The pharmaceutical composition according to claim 3, wherein the content by weight of the poly(lactide-co-glycolide) in the triptorelin microspheres is 60-98.9%.

5. The pharmaceutical composition according to claim 4, wherein the content by weight of triptorelin or the salt thereof in the triptorelin microspheres is 1-30%, and the content by weight of the poly(lactide-co-glycolide) in the triptorelin microspheres is 60-98.9%, and the content by weight of glucose in the triptorelin microspheres is 0.1-10%.

6. The pharmaceutical composition according to claim 5, wherein the content by weight of triptorelin or the salt thereof in the triptorelin microspheres is 2-20%, and the content by weight of the poly(lactide-co-glycolide) in the triptorelin microspheres is 70-97.5%, and the content by weight of glucose in the triptorelin microspheres is 0.5-10%.

7. The pharmaceutical composition according to claim 6, wherein the content by weight of triptorelin or the salt thereof in the triptorelin microspheres is 2-20%, and the content by weight of the poly(lactide-co-glycolide) in the triptorelin microspheres is 75-97.5%, and the content by weight of glucose in the triptorelin microspheres is 0.5-5%.

8. The pharmaceutical composition according to claim 7, wherein the content by weight of triptorelin or the salt thereof in the triptorelin microspheres is 5-15%, and the content by weight of the poly(lactide-co-glycolide) in the triptorelin microspheres is 83-94.5%, and the content by weight of glucose in the triptorelin microspheres is 0.5-2%.

9. The pharmaceutical composition according to claim 8, wherein the content by weight of triptorelin or the salt thereof in the triptorelin microspheres is 10%, and the content by weight of the poly(lactide-co-glycolide) in the triptorelin microspheres is 89%, and the content by weight of glucose in the triptorelin microspheres is 1 %.

10. The pharmaceutical composition according to any of claims 1 to 8, wherein the salt of triptorelin is acetates.

11. The pharmaceutical composition according to claims 1 to 8, wherein the triptorelin microspheres is prepared by a double emulsion-solvent evaporation process, with glucose being added into an inner water phase.

12. The pharmaceutical composition according to claim 10, wherein the molar ratio of lactide to glycolide in the poly(lactide-co-glycolide) is within a range from 90:10 to 40:60, or 75:25 to 40:60, or 50:50 .

13. The pharmaceutical composition according to claim 10, wherein the intrinsic viscosity of the poly(lactide-co-glycolide) is 0.10-0.70 dL/g, preferably 0.15-0.50 dL/g, and more preferably 0.20-0.35 dL/g, wherein the intrinsic viscosity is measured as follows: preparing an 0.5% (w/v) solution of PLGA in chloroform, and determining the intrinsic viscosity of PLGA at 30°C using a Cannon-Fenske glass capillary viscometer.

14. The pharmaceutical composition according to claim 10, wherein the weight average molecular weight of the poly(lactide-co-glycolide) is 5,000-100,000 Dalton, preferably 10,000-7,5000 Dalton, and more preferably 15,000-40,000 Dalton.

15. The pharmaceutical composition according to claim 1 to 14 wherein the microspheres can be made into the form of sterile powder comprises the composition of triptorelin microspheres according to claims 1 to 14 and mannitol.

16. The pharmaceutical composition according to any claims of 1 to 8 for use in the treatment of prostate cancer, sexual precocity, adenomyosis, female infertility, or hysteromyoma.

## Patentansprüche

1. Eine pharmazeutische Zusammensetzung von Triptorelin-Mikrokugeln mit anhaltender Freisetzung, wobei die Triptorelin-Mikrokugeln Triptorelin oder ein Salz davon, Polylactid-co-Glycolid und Glucose umfassen.

2. Die pharmazeutische Zusammensetzung nach Anspruch 1, wobei der Gewichtsanteil von Glucose in den Triptorelin-Mikrokugeln 0,1-10% oder 0,5-10% oder 0,5-5% oder 0,5-2% oder 1% beträgt.

3. Die pharmazeutische Zusammensetzung nach Anspruch 2, wobei der Gewichtsanteil von Triptorelin oder einem Salz davon in den Triptorelin-Mikrokugeln 1-30% beträgt.

4. Die pharmazeutische Zusammensetzung nach Anspruch 3, wobei der Gewichtsanteil von Triptorelin oder einem Salz davon in den Triptorelin-Mikrokugeln 60-98,9% beträgt.

5. Die pharmazeutische Zusammensetzung nach Anspruch 4, wobei der Gewichtsanteil von Triptorelin oder einem Salz davon in den Triptorelin-Mikrokugeln 1-30% beträgt, der Gewichtsanteil von Polylactid-co-Glycolid in den Triptorelin-Mikrokugeln 60-98,9% beträgt und der Gewichtsanteil von Glucose in den Triptorelin-Mikrokugeln 0,1-10% beträgt.

6. Die pharmazeutische Zusammensetzung nach Anspruch 5, wobei der Gewichtsanteil von Triptorelin oder einem Salz davon in den Triptorelin-Mikrokugeln 2-20% beträgt, der Gewichtsanteil von Polylactid-co-Glycolid in den Triptorelin-Mikrokugeln 70-97,5% beträgt und der Gewichtsanteil von Glucose in den Triptorelin-Mikrokugeln 0,5-10% beträgt.

7. Die pharmazeutische Zusammensetzung nach Anspruch 6, wobei der Gewichtsanteil von Triptorelin oder einem Salz davon in den Triptorelin-Mikrokugeln 2-20% beträgt, der Gewichtsanteil von Polylactid-co-Glycolid in den Triptorelin-Mikrokugeln 75-97,5% beträgt und der Gewichtsanteil von Glucose in den Triptorelin-Mikrokugeln 0,5-5% beträgt.

8. Die pharmazeutische Zusammensetzung nach Anspruch 7, wobei der Gewichtsanteil von Triptorelin oder einem Salz davon in den Triptorelin-Mikrokugeln 5-15% beträgt, der Gewichtsanteil von Polylactid-co-Glycolid in den Triptorelin-Mikrokugeln 83-94,5% beträgt und der Gewichtsanteil von Glucose in den Triptorelin-Mikrokugeln 0,5-2% beträgt.

9. Die pharmazeutische Zusammensetzung nach Anspruch 8, wobei der Gewichtsanteil von Triptorelin oder einem Salz davon in den Triptorelin-Mikrokugeln 10% beträgt, der Gewichtsanteil von Polylactid-co-Glycolid in den Triptorelin-Mikrokugeln 89% beträgt und der Gewichtsanteil von Glucose in den Triptorelin-Mikrokugeln 1% beträgt.

10. Die pharmazeutische Zusammensetzung nach einem der Ansprüche 1 bis 8, wobei das Salz von Triptorelin ein Acetat ist.

11. Die pharmazeutische Zusammensetzung nach einem der Ansprüche 1 bis 8, wobei die Triptorelin-Mikrokugeln durch einen Doppelemulsions-Lösungsmittel-Verdampfungsprozess hergestellt werden, wobei Glucose einer inneren Wasserphase zugesetzt wird.

12. Die pharmazeutische Zusammensetzung nach Anspruch 10, wobei das molare Verhältnis von Lactid zu Glycolid im Polylactid-co-Glycolid in einem Bereich von 90:10 bis 40:60 oder 75:25 bis 40:60 oder 60:40 bis 40:60 oder bei 50:50 liegt.

13. Die pharmazeutische Zusammensetzung nach Anspruch 10, wobei die intrinsische Viskosität des Polylactid-co-Glycolids 0,10-0,70 dL/g, bevorzugt 0,15-0,50 dL/g, und mehr bevorzugt 0,20-0,35 dL/g beträgt, wobei die intrinsische Viskosität wie folgt gemessen wird: Herstellen einer 0,5% (Gewicht/Volumen) Lösung von PLGA in Chloroform und Bestimmen der intrinsischen Viskosität von PLGA bei 30°C mit einem Cannon-Fenske Glaskapillaren-Viskosimeter.

14. Die pharmazeutische Zusammensetzung nach Anspruch 10, wobei das mittlere Molekulargewicht des Polylactid-co-Glycolids 5000-100000 Dalton, bevorzugt 10000-75000 Dalton, und mehr bevorzugt 15000-40000 Dalton beträgt.

15. Die pharmazeutische Zusammensetzung nach den Ansprüchen 1 bis 14, wobei die Mikrokugeln in die Form eines sterilen Pulvers gebracht werden können, wobei dieses die Zusammensetzung für Triptorelin-Mikrokugeln nach den Ansprüchen 1 bis 14 und Mannitol umfasst.

16. Die pharmazeutische Zusammensetzung nach den Ansprüchen 1 bis 8 zur Verwendung für die Behandlung von Prostatakrebs, sexueller Frühreife, Adenomyose, weiblicher Unfruchtbarkeit oder Hysteromyomen.

## Revendications

1. Composition pharmaceutique de microsphères de triptoréline à libération prolongée, les microsphères de triptoréline comprenant de la triptoréline ou l'un de ses sels, du poly(lactide-co-glycolide) et du glucose.

2. Composition pharmaceutique selon la revendication 1, où la teneur en masse de glucose dans les microsphères de triptoréline est comprise entre 0,1 et 10 %, ou entre 0,5 et 10 %, ou entre 0,5 et 5%, ou encore entre 0,5 et 2 %, ou est égale à 1 %.

3. Composition pharmaceutique selon la revendication 2, où la teneur en masse de triptoréline ou de son sel dans les microsphères de triptoréline est comprise entre 1 et 30 %.

4. Composition pharmaceutique selon la revendication 3, où la teneur en masse de triptoréline ou de son sel dans les microsphères de triptoréline est comprise entre 60 et 98,9 %.

5. Composition pharmaceutique selon la revendication 4, où la teneur en masse de triptoréline ou de son sel dans les microsphères de triptoréline est comprise entre 1 et 30 %, la teneur en masse du poly(lactide-co-glycolide) dans les microsphères de triptoréline est comprise entre 60 et 98,9 %, et la teneur en masse de glucose dans les microsphères de triptoréline est comprise entre 0,1 et 10 %.

6. Composition pharmaceutique selon la revendication 5, où la teneur en masse de triptoréline ou de son sel dans les microsphères de triptoréline est comprise entre 2 et 20 %, la teneur en masse du poly(lactide-co-glycolide) dans les microsphères de triptoréline est comprise entre 70 et 97,5 %, et la teneur en masse de glucose dans les microsphères de triptoréline est comprise entre 0,5 et 10 %.

7. Composition pharmaceutique selon la revendication 6, où la teneur en masse de triptoréline ou de son sel dans les microsphères de triptoréline est comprise entre 2 et 20 %, la teneur en masse du poly(lactide-co-glycolide) dans les microsphères de triptoréline est comprise entre 75 et 97,5 %, et la teneur en masse de glucose dans les microsphères de triptoréline est comprise entre 0,5 et 5 %.

8. Composition pharmaceutique selon la revendication 7, où la teneur en masse de triptoréline ou de son sel dans les microsphères de triptoréline est comprise entre 5 et 15 %, et la teneur en masse du poly(lactide-co-glycolide) dans les microsphères de triptoréline est comprise entre 83 et 94,5 %, et la teneur en masse de glucose dans les microsphères de triptoréline est comprise entre 0,5 et 2 %.

9. Composition pharmaceutique selon la revendication 8, où la teneur en masse de triptoréline ou de son sel dans les microsphères de triptoréline est de 10 %, la teneur en masse du poly(lactide-co-glycolide) dans les microsphères de triptoréline est de 89 %, et la teneur en masse de glucose dans les microsphères de triptoréline est de 1 %.

10. Composition pharmaceutique selon l'une quelconque des revendications 1 à 8, où le sel de triptoréline est un acétate.

11. Composition pharmaceutique selon les revendications 1 à 8, où les microsphères de triptoréline sont préparées par un double procédé d'émulsion et d'évaporation de solvant, le glucose étant ajouté dans une phase aqueuse interne.

12. Composition pharmaceutique selon la revendication 10, où le rapport molaire lactide sur glycolide dans le poly(lactide-co-glycolide) est compris dans un intervalle allant de 90:10 à 40:60, ou de 75:25 à 40:60, ou est égal à 50:50.

13. Composition pharmaceutique selon la revendication 10, où la viscosité intrinsèque du poly(lactide-co-glycolide) est comprise entre 0,10 et 0,70 dL/g, préférentiellement entre 0,15 et 0,50 dL/g, et plus préférentiellement entre 0,20 et 0,35 dL/g, où la viscosité intrinsèque est mesurée de la façon suivante : préparation d'une solution à 0,5 % en masse par volume de PLGA dans le chloroforme, et détermination de la viscosité intrinsèque du PLGA à 30 °C à l'aide d'un viscosimètre à capillaire de verre de Cannon-Fenske.

14. Composition pharmaceutique selon la revendication 10, où la masse moléculaire moyenne en poids du poly(lactide-co-glycolide) est comprise entre 5000 et 100 000 daltons, préférentiellement entre 10 000 et 75 000 daltons, et plus préférentiellement entre 15 000 et 40 000 daltons.

15. Composition pharmaceutique selon les revendications 1 à 14, où les microsphères peuvent être façonnées en forme de poudre stérile comprenant la composition de microsphères de triptoréline selon les revendications 1 à 14 et du mannitol.

16. Composition pharmaceutique selon l'une quelconque des revendications 1 à 8 pour utilisation dans le traitement du cancer de la prostate, de la précocité sexuelle, de l'adénomyose, de l'infertilité féminine ou de l'hystéromyome.
